Europäisches Patentamt

European Patent Office

Office européen des brevets

(11) Publication number: **0 210 970**
**A2**

## (12) EUROPEAN PATENT APPLICATION

(21) Application number: 86850265.9

(22) Date of filing: 23.07.86

(51) Int. Cl.⁴ **A61K 49/02** , A61K 43/00 , A61K 39/395 , C07K 15/00 , C12P 21/00 , C12N 5/00 , C12N 15/00 , //(C12P21/00,C12R1:91)

A request for correction of the description has been filed pursuant to Rule 88 EPC. A decision on the request will be taken during the proceedings before the Examining Division (Guidelines for Examination in the EPO, A-V, 2.2).

(30) Priority: 25.07.85 US 759583

(43) Date of publication of application:
04.02.87 Bulletin 87/06

(84) Designated Contracting States:
AT BE CH DE FR GB IT LI LU NL SE

(71) Applicant: REGENTS OF THE UNIVERSITY OF MINNESOTA
100 Church Street Southeast
Minneapolis, Minnesota 55455(US)

(72) Inventor: Vessella, Robert L.
13026 Euclid Avenue
Apple Valley, MN 55124(US)
Inventor: Lange, Paul
7410 Timber Ridge Court
Minnetonka, MN 55345(US)

(74) Representative: Wallin, Bo-Göran et al
AWAPATENT AB Box 5117
S-200 71 Malmö(SE)

(54) Detection, imaging and therapy of renal cell carcinoma with monoclonal antibodies.

(57) Three monoclonal antibodies which are highly reactive with human renal cell carcinoma (RCC) were generated by hybridoma techniques and characterized a cell-binding ELISA and immunohistological examination of tumor, normal and fetal tissue sections. The radiolabeled antibodies selectively localized to RCC xenografts and provided high resolution images of the xenographs without the need for background subtraction. The localized, labelled antibodies also produced a regression in the size of the tumors.

# DETECTION, IMAGING, AND THERAPY OF RENAL CELL CARCINOMA WITH MONOCLONAL ANTIBODIES

## Background of the Invention

Renal cell carcinoma (RCC) is a common malignancy, with approximately 20,000 new cases reported annually. In its early stages, RCC is often insidious and asymptomatic with the result that approximately 60% of RCC patients have known or occult metastasis on initial diagnosis. For these patients there is no effective treatment. In those patients where excision of the malignancy is warranted, pathological grading remains difficult and subjective. In fact, although electron microscopic studies suggest the convoluted proximal tubule cell may be the site of origin, confirmatory evidence is lacking and the issue remains controversial. Also, sometimes RCC has an unusual natural history: i.e., RCC has the highest rate of spontaneous regression of any human tumor and some patients live in apparent symbiosis with their metastatic tumor(s) for many years. Thus, the study of RCC can yield important information relating to the host-tumor relationship. Also, in contrast to many of the solid human tumors (e.g., those of the germ cells, prostate, colon, and lung), there are presently no immunodiagnostic assays for RCC, which is an epithelial tissue tumor. Furthermore, RCC has no putative markers to monitor for recurrence or aid in the diagnosis of primary disease.

The application of hybridoma techniques to the study of human solid tumors has resulted in the generation of monoclonal antibodies (Mabs) that recognize tumor-associated antigens. Mabs reactive with solid tumors have demonstrated wide degrees of restriction, including some which demonstrate high levels of tumor specificity, although it has been hypothesized that complete tumor specificity may not be found. Nevertheless, some of these Mabs have clinical potential; they often recognize tumor-associated antigens which distinguish a given tumor type from its counterpart normal tissue, other normal tissues, fetal tissues, and other tumor types either due to a quantitative or qualitative difference in antigen expression and/or secretion.

Monoclonal antibodies which exhibit some degree of binding specificity to one or more classes of RCC have been investigated. In Proc. Natl. Acad. Sci. USA, 78, 5122 (1981), R. Ueda et al. describe several Mabs which recognized antigenic determinants common to the RCC cell membrane with various degrees of restriction to normal cells or other tumors. All of these Mabs recognized one or more organelle(s) of the normal kidney and their use in immunopathological studies have contributed to the understanding of renal differentiation and RCC cellular origin. Scharfe et al. recently reported the generation RCC-reactive Mabs that were preferential for low-grade tumors and did not react with normal renal structures. [AUA Annual Meeting, 1984, Abstract 104] However, all of the Mabs recognized antigenic determinants co-expressed by normal colon, thus limiting their diagnostic and therapeutic utility.

Potential applications of RCC-reactive Mabs are in the detection, e.g., by photographic visualization, of occult metastatic disease or identification of questionable renal masses through radioimmunoscintigraphy (RIS). Precise visualization of tumor cells can improve the precision with which treatment such as radiation can be delivered to the tumor site, while the correct identification of the tumor variety is necessary to insure the choice of the most effective treatment.

The detection of tumors by scintillation scanning has been attempted for many years with only limited success. Early efforts involved nonspecific tumor-seeking radiopharmaceuticals such as gallium and bleomycin-cobalt, but lack of specificity limited their usefulness. Other investigators explored radioimmunoscintigraphy (RIS), the combination of nuclear medicine techniques with tumor "specific" xenogenic antibodies. Results did improve modestly, in part because of advances in radiopharmacology and computer science, but still success was insufficient for widespread clinical implementation. The polyclonal antibodies directed to membrane-associated antigens continued to lack high specificity and those which were specific for soluble tumor markers yielded images which lacked sensitivity due to high levels of background noise.

The production of monoclonal antibodies increased the expectation that RIS would become a valuable clinial tool, although to date the results have been mixed. RIS studies with radiolabeled Mabs which recognized soluble tumor markers provided modest increases in sensitivity. Mabs have been generated to tumor membrane antigens but they often reacted with antigenic determinants shared by other tissues and RIS of tumors using these Mabs improved the images but significant problems remain. For example, visualization of tumor xenografts weighing less than 100 mg has been infrequent. Moreover, in many previous studies, the amount of radiolabel within the xenograft - (on a per weight basis) was only marginally above the levels in the blood or normal tissue. Recent efforts to improve the quality of the tumor scan with available Mabs have involved a variety of manipulations including sophisticated computer-assisted subtraction techniques, such as those disclosed by M.D. Goldenberg in U.S. Pat. 4,460,561. The use of antibody fragments, or radioisotopes other than iodine have also been attempted, but have been only partially successful.

Thus, a substantial need exists for methods to produce monoclonal antibodies (Mabs) that can react with RCC while not exhibiting substantial reactivity with normal cells or with other types of malignancies. A need also exists for effective methods to employ such antibodies for the diagnosis, visualization and treatment of RCC.

Brief Description of the Invention

The present invention is directed to monoclonal antibodies (Mabs) which are highly specific or preferential to renal cell carcinoma (RCC). The monoclonal antibodies were produced by propagating hybridomas formed by fusing a myeloma cell with a cell capable of producing antibodies against antigenic RCC material, including (1) homogenates of surgical specimens (RCC or fetal kidney), (2) RCC cell lines and (3) cell membranes of RCC cell lines. The antibodies produced by the hybridomas were then isolated and characterized. For the selection of RCC preferential Mabs, two complementary screening systems were used -a cell-binding ELISA incorporating multiple tissue culture cell lines and an evaluation of the immunohistochemical staining patterns of frozen surgical specimens (normal, malignant and fetal tissues).

One Mab (D5D) was found to react only with RCC cell lines and can recognize an epitope restricted to poorly-moderately differentiated, but not well differentiated RCC or any other tissues. Another Mab (A6H) appears to have a higher proclivity for granular-, rather than clear-, cell histology in RCC surgical specimens and reacts strongly at dilutions of ≤1ng/ml to many of the RCC cell lines. It also reacts with the proximal tubules of normal kidney and marginally with two non-RCC cell lines. A third Mab (C5H) recognizes an epitope shared by many tumors, including RCC, and the normal kidney glomerulus. All other normal tissues are nonreactive to the present Mabs.

The three Mabs were labeled with I-125 and I-131 and used for detecting and imaging RCC tumor xenografts in nude mice. Another radiolabeled Mab, reactive to alpha feto protein (AFP), was used as a control. A majority of the mice bore one or two other xenografts (non-RCC) that served as internal controls. Clear images (pinhole collimator, 20-40 uCi I-131 Mab) were obtained of the RCC xenografts (≥ 10 mg) at 5, 24, and 48 hours without the need for background subtraction. After imaging, the mice were sacrificed and the radioactivity of various tissues and organs measured. A useful indicator of specificity and imaging potential is the ratio between accumulation of labeled Mab in the target tissue compared to the accumulation in the blood pool. The RCC xenograft cpm/g to blood cpm/g ratios were within the range of about from 4 to 60:1 while the ratios for normal tissues and control tumors to blood was usually ≤1:1. The RCC xenograft to blood ratio was ≤1:1 when radiolabeled AFP Mab was used. The immunostaining of tissues by the Mabs in vitro can predict the degree of localization of the radiolabeled Mabs in vivo. Therefore, specificity of immunostaining of tissues provides a method for preselecting Mabs for radiolocalization from a library of potentially useful antibodies.

To demonstrate the effectiveness of radioimmunotherapy using the present radiolabeled Mabs, mice bearing RCC xenografts received (1) no treatment, (2) non-radiolabeled A6H, (3) I-131-labeled control IgG, MAB (I-131-AFP) (100 uCi), and (4) I-131-labeled A6H (100 uCi). Mice receiving no treatment, non-labeled A6H or labeled control MAB exhibited rapid progression of disease, while mice treated with I-131-labeled A6H exhibited substantial reduction in tumor size. Therefore, the preselected Mabs of the present invention localized in target RCC tumor tissue in amounts sufficient for imaging without background subtraction or extensive computer processing. In addition, the present Mabs can afford a means for the effective radioimmunotherapy of RCC xenografts with substantial arrest of growth and reduction in tumor size.

Detailed Description of the Invention

A. Monoclonal Antibodies

The general techniques for producing monoclonal antibodies are based on the fusion of spleen lymphocytes with malignant cells (myelomas) of bone marrow primary tumors [C. Milstein, Sci. Am., 243 , 66 (1980)]. The methods yield a hybrid cell line, arising from a single fused cell hybrid, or clone, which possesses characteristics of both the lymphocytes and myeloma cell lines. Like the lymphocytes (taken from animals primed with sheep red blood cells as antigens), the fused hybrids or hybridomas secrete a single type of antibody (immunoglobulin) specific to the antigen. Moreover, like the myeloma cell lines, the hybrid cell lines are immortal. Whereas antisera derived from vaccinated animals are variable mixtures of antibodies which cannot be identically reproduced, the single type of immunoglobulin secreted by a hybridoma is specific to one and only one antigenic determinant on the antigen, a complex molecule having a multiplicity of antigenic molecular substructures, or determinants. For instance, if the antigen is a protein, an antigenic determinant may be one of the many peptide sequences within the entire protein molecule. Hence, monoclonal antibodies raised against a single antigen may be distinct from each other depending on the determinant that induced their formation. However, all of the antibodies produced by a given clone are identical. Furthermore, preferred hybridoma cell lines can be reproduced indefinitely, are easily propagated in vitro or in vivo, and yield monoclonal antibodies in extremely high concentration.

## 1. Antigens

Methods for the production of monoclonal antibodies are generally applicable and have been used to produce antibodies to a wide variety of antigens.

Antigenic preparations useful to induce the production of anti-renal tumor cell antibodies in primed animals include RCC cell lines, fetal kidney homogenates and RCC tumor homogenates. The binding specificity of a given monoclonal antibody to a target determinant can be enhanced in some cases by tandem immunization protocols, wherein the animal to be primed is injected sequentially with phenotypically similar but genotypically dissimilar tumors, tissues or cell cultures. This strategy can promote a hyperimmune response to antigens shared among the cells, but with minimal reinforcement of the allogeneic antigens. This approach can increase the chances of developing specific tumor-reactive or "restricted" monoclonal antibodies. See R.L. Vessella et al., Fed. Proc., 42, 401 (1983).

## 2. Somatic Cells

Somatic cells with the potential for producing antibody, and in particular B cells, are suitable for fusion with a B-cell myeloma line. Those antibody-producing cells undergoing mitosis fuse preferentially. Lymph nodes and spleens of the primed animals are convenient lymphatic organs for use in the present fusion system. Once-primed or hyperimmunized, animals can be used as a source of antibody-producing lymphocytes. Mouse and rat lymphocytes give a higher percentage of stable fusions with the mouse myeloma lines described below. However, the use of rabbit, human primate and frog cells is also possible. In the preferred embodiments, hyperimmunized mouse spleen cells are used to make the fused cell hybrids.

## 3. Myeloma Cells

Specialized myeloma cell lines have been developed from lymphocyte tumors for use in hybridoma-producing fusion procedures [G. Kohler and C. Milstein, Eur. J. Immunol., 6, 511 (1976); M. Shulman et al., Nature, 276, 269 (1978)]. The cell lines have been developed to facilitate the selection of fused hybridomas among unfused and similarly indefinitely self-propagating myeloma cells by using myelomas with enzyme deficiencies that render them incapable of growing in certain selective media that support the growth of hybridomas. Furthermore, myeloma cell lines incapable of producing light or heavy immunoglobulin chains or those deficient in antibody secretion mechanisms are used. A third reason for selection of cell lines is for their suitability and efficiency for fusion.

Several myeloma cell lines may be used for the production of fused cell hybrids, including P3/X63-Ag 8.653, P3/NSI/1-Ag 4-1, Sp2/0-Ag14 and S194/5.XXO.BU.1. The P3/X63-Ag 8 and P3/NSI/1-Ag 4-1 cell lines have been described by Kohler and Milstein [Eur. J. Immunol., 6, 511 (1976)] and by J. Kearney et al., J. Immunol., 123, 1548 (1979). Shulman et al. [Nature, 276, 269 (1978) developed the Sp2/0-Ag14 myeloma line. The S194/5.XXO.BU.1 myeloma line was reported in an article by Trowbridge [J. Exp. Med., 148, 313 - (1979)]. In the examples of the present invention, P3/NSI/1-Ag 4-1 and P3/X63-Ag 8.653 (derived from BALB/c mice) are the preferred cell lines.

## 4. Fusion

Methods for generating hybrids of antibody-producing spleen or lymph node cells and myeloma cells usually comprise mixing somatic cells with myeloma cells in a 10:1 proportion - (though the proportion may vary from 20:1 to 1:1), respectively, in the presence of an agent or agents that promote the fusion of cell membranes. It is preferred that the same species of animal serve as the source of the somatic and myeloma cells used in the fusion procedure. Fusion methods have been described by Kohler and Milstein in Nature, 256, 495 (1975) and Eur. J. Immunol., 6, 511 (1976), and by Gefter et al. in Somatic Cell Genet, 3, 231 - (1977). The fusion-promoting agents used by those investigators were Sendai virus and polyethylene glycol (PEG), respectively. The fusion procedure of the example of the present invention is that of V. Oi et al., as set forth in Selected Methods in Cellular Immunology, W.H. Freeman and Co., San Francisco (1980) at pages 351-372, the disclosure of which is incorporated by reference herein. This method employed PEG as the fusion agent.

## 5. Isolation of Clones and Antibody Detection and Production

Generally the selection of fused cell hybrids is accomplished by culturing the cells in media that support the growth of hybridomas but prevent the growth of the unfused myeloma cells which normally would go on dividing indefinitely. In the example of the present invention, myeloma cells lacking hypoxanthine phosphoribosyl transferase - (HPRT⁻) were used. These cells are selected against in hypoxanthine/aminopterin/thymidine (HAT) medium, a medium in which the fused cell hybrids survive due to the HPRT-positive genotype of the spleen cells. The use of myeloma cells with different genetic deficiencies (e.g., other enzyme deficiencies, drug sensitivities, and the like) that can be selected against in media supporting the growth of genotypically competent hybrids is also possible.

Several weeks can be required to selectively culture the fused cell hybrids. Early in this time period, it is necessary to identify those hybrids which produce the desired antibody so that they may be subsequently cloned and propagated. The detection of antibody-producing hybrids can be achieved by any one of several standard assay methods, including enzyme-linked immunoassay and radioimmunoassay techniques which have been described in the literature [R. Kennet, T. McKearn and K. Bechtol (editors), Monoclonal Antibodies, Hybridomas: A New Dimension In Biological Analyses, Plenum Press, New York (1980) at pages 376-384].

The detection method used in the example of the present invention was a cell-binding enzyme-linked immunoassay (ELISA) employing an avidin-biotin immunoperoxidase system. This method could be used to detect monoclonal antibodies in hybridoma supernatant media fluids, ascites fluid or in preparations comprising the purified antibodies.

Once the desired fused cell hybrids have been selected and cloned into individual antibody-producing cell lines, each cell line may be propagated in either of two standard ways. A sample of the hybridoma can be injected into a histocompatible animal of the type that was used to provide the somatic and myeloma cells for the original fusion. The injected animal develops tumors secreting the specific monoclonal antibody produced by the fused cell hybrid. The body fluids of the animal, such as serum or ascites fluid, can be tapped to provide monoclonal antibodies in high concentration. Alternatively, the individual cell lines may be propagated in vitro in laboratory culture vessels. The culture medium, also containing high concentrations of a single specific monoclonal antibody, can be harvested by decantation, filtration or centrifugation, and the antibody isolated therefrom.

## 6. Isotyping of Monoclonal Antibodies

Immunoglobulin isotype and subclass can be determined by the Ouchterlony immunoprecipitation technique using rabbit anti-mouse immunoglobulin subclass antibodies (Litton Bionetics, Kensington, MA).

## 7. Characterization of the Monoclonal Antibodies

### (a) Specificity Testing

The specific binding reactivity of the monoclonal antibodies of the present invention can be measured by comparing the reactivity of the monoclonal antibodies with established renal cell carcinoma lines and with other established human cancer cell lines by ELISA procedures such as those employed to isolate the antibody-producing cell lines.

### (b) Immunopathology

To determine whether the reactivity patterns of tissue cultured cells were an accurate reflection of in vivo antigen expression, the immunohistochemical reactivity of Mabs were analyzed against a tissue panel consisting of surgical specimens of human tumors, normal, and fetal tissues. Tissue specimens were diced following excision, frozen and sectioned with a cryostat or microtome. The sections were fixed and incubated with the purified monoclonal antibodies or with fresh hybridoma culture supernatants. The tissue sections were then washed, overlaid with an antibody labeled with a fluorescent label, washed and contacted with a fluorescent-labeled antibody to the overlaid antibody. The sections were evaluated by means of an epi-fluorescence and phase microscope.

Enzyme-linked immunoassays were performed employing enzyme-labeled antibodies by standard techniques, such as those set forth in U.S. Pat. 4,228,237, the disclosure of which is incorporated herein by reference.

## 8. Labeling

The monoclonal antibodies may be labeled by any of several techniques known to the art. A wide range of labeling techniques are disclosed in Feteanu et al., "Labeled Antibodies in Biology and Medicine", pages 214-309 (McGraw-Hill Int. Book Co., New York (1978)). The introduction of various metal radioisotopes may be accomplished according to the procedures of D. J. Hnatowich et al., Science, 220, 613 (1983); Wagner et al., J. Nucl. Med., 20, 428 (1979); Sundberg et al., J. Med. Chem., 17, 1304 (1974); and Saha et al., J. Nucl. Med., 6, 542 (1976).

Among the radioisotopes used, x-ray-emitters, gamma-emitters, positron-emitters, and fluorescence-emitters are suitable for localization and/or therapy, while beta-emitters and alpha-emitters can also be used for therapy. Suitable radioisotopes for labeling antibodies include Iodine-131, Iodine-123, Iodine-125, Iodine-126, Iodine-133, Bromine-77, Indium-111, Indium-113m, Gallium-67, Gallium-68, Ruthenium-95, Ruthenium-97, Ruthenium-103, Ruthenium-105, Mercury-107, Mercury-203, Rhenium-99m, Rhenium-105, Rhenium-101, Scandium-47, Tellurium-121m, Tellurium-122m, Tellurium-125m, Thulium-165, Thulium-167, Thulium-168, Technetium-99m, Copper 67, Fluorine-18, Yttrium 90, Gold 199, Palladium 100, Bismuth 217 and Antimony 211. Preferred radioisotopes for labeling antibodies employed for tumor imaging include Iodine 125, 131 or 123, Indium 111, Ruthenium 97, Copper 67 and Technicium 99. Preferred radioisotopes for labeling antibodies to form therapeutic agents include Iodine 131, Yttrium 90, Gold 199, Palladium 100, Copper 67, Bismuth 217 and Antimony 211. The halogens can be used more or less interchangeably as labels since halogen-labeled antibodies and/or normal immunoglobulins would have substantially the same kine tics and distribution and similar metabolism. Preferably the radioisotope will emit in the 10-5,000 kev range, more preferably 100-500 kev.

One useful labeling technique involves labeling with either Iodine-131 (I-131) or Iodine-125 (I-125) using an oxidative procedure wherein a mixture of radioactive potassium or sodium iodide and the antibody is treated with chloramine-T (N-chloro-p-toluene sulfonamide sodium salt) e.g., as reported by Greenwood et al., in Biochem. J., 89 , 114 - (1963) and modified by McConahey et al., in Int. Arch. Allergy Appln. Immunol., 29, 185 (1969).

In general, it is desirable to introduce as high a proportion of radiolabel as possible into the antibody molecule without destroying its immunospecificity. For each labeled antibody, the extent of the reduction in labeled antibody-antigen binding was established by standard competitive cell binding assays. Preferably, at least about 30% of the initial binding activity of the antibody was preserved when 125-I or 131-I were used as the labels.

Chemotherapeutic agents or cytotoxins can also be bound to the antibodies, as required, by techniques known to the art.

## B. Radioimmunoscintigraphy of Tumor Xenografts

Human RCC and control tumor xenographs were established in test animals by subcutaneous inoculation of the test animals (e.g., mice) with fresh tumor specimens or with cells from human tumor cell lines established by known procedures, such as those disclosed by N.J. Vogelzang et al., in Cold Spring Harbor Conferences on Cell Proliferation, 10, Teratocarcinoma Stem Cells, pp. 607-614 (1983), the disclosure of which is incorporated by reference herein. Preferably, the majority of the mice studied bore at least two xenografts: (a) an RCC and one or two non-RCC xenografts or (b) two RCC xenografts. The non-RCC xenografts served as internal controls.

Biodistribution studies were performed on all of the mice bearing xenografts or skin abscesses and were designed to evaluate (1) the specificity of Mab tissue distribution using both RCC and non-RCC xenograft combinations, (2) the changes in tissue:blood distribution (T:B) ratios with time after injection, (3) the influence of xenograft size on the T:B ratios, and (4) whether areas of acute tissue inflammation non-specifically accumulated radiolabeled Mabs.

For studies of radiolabel biodistribution which did not include scintigraphy, mice were injected intravenously with about 1-2 uCi of the labeled antibody. Mice which were to be imaged by RIS received about 20-40 uCi of the labeled antibody, but always received equivalent doses of the imaging Mab and the control Mab per study. Immediately following scintigraphy, the mice were sacrificed and samples of the xenograft(s), the other tissue types, blood, and urine were collected. The samples were weighed, the amount of radiolabel in each determined, and from these data (cpm/mg) the T:B or T:T (tumor:blood or tissue) ratios were calculated. These ratios are referred to as the biodistribution index. Mice with skin abscesses were included in one series to determine the affects of acute inflammation on RIS. Initial RIS studies were performed at 5 hours, 1 and 2 days after injection of the radiolabeled Mab. Thereafter, images were obtained 2 days after injection of radiolabel.

Scintigraphy can be performed using commercially-available photoscanning equipment, such as a Siemens Pho Gamma V camera equipped with a pinhole collimator. Imaging and sampling was done in a blinded manner; investigators performing the imaging were not aware of the xenograft types (RCC or a control tumor) or of which radiolabeled Mab was injected. Ten-thousand count images were normally acquired over a 3-to 5-minute period and data were stored in a computer for further analysis and production of color imagings. Background subtraction techniques were not utilized.

## C. Immunotherapy

The radiolabeled tumor-specific Mabs of the present invention can be employed to halt RCC growth and to induce regression and/or elimination of the tumor mass. For example, the biodistribution index of a given labeled Mab can be used to estimate the amount of radioactivity which can be delivered to the tumor site per dose of Mab. Preferably, the appropriate Mab can be introduced into the subject and delivered to the tumor site by intravenous injection or infusion in a pharmaceutically-acceptable liquid carrier such as a physiological salt solution. Alternatively, a solution or suspension of the labeled Mab can be perfused directly into malignant epithelial tissue, a method which may be preferred in cases in which the RCC has not metathesized. Thus, the labeled Mab of the present invention can permit the simultaneous treatment and monitoring of tumor size and progression. For a general discussion of the treatment of tumors with radiolabeled monoclonal antibodies, see U.S. Pat. 4,348,376, the disclosure of which is incorporated by reference herein.

The invention will be further described by reference to the following detailed examples.

## Example 1

### A. Hybridoma Techniques

Barrier-reared BALB/c mice were immunized with a variety of antigenic preparations [RCC cell lines ($10^7$ cells i.p. in saline x 6), fetal kidney homogenates (10% w/v i.p. in saline x 4) and/or RCC tumor homogenates (10% w/v i.p. in saline x 5) using an immunization schedule consisting of five (5) intraperitoneal injections over a period of 2-3 months, with a final injection three (3) days before sacrifice. The mouse spleen cells were fused with either P3-NS1-Ag4-1 or P3X63-Ag8.653 (a gift of Dr. Kearney, University of Alabama) mouse myeloma cells, by the method of Oi et al., *supra*. After specificity testing, as described herein below, cultures were cloned three times by the limiting dilution technique (20% spent media from myeloma cultures as growth supplements along with neonatal thymus cells as a feeder layer) and the Mab-producing hybridomas were subsequently grown as ascites tumors in pristane primed BALB/c mice.

In some studies the immunization protocol was modified to promote Mabs which recognize phenotypic antigens common among genetically dissimilar tissues. This protocol, which we referred to as tandem immunization comprises the sequential injection of a plurality of phenotypically similar, genotypically dissimilar, antigen preparations, tissue homogenates or cell lines as disclosed by R.L. Vessella et al., in Fed. Proc., 42, 401 (1983).

## B. Specificity Testing of Hybridomas

### (i) Cell Binding ELISA

Antibodies were tested for reactivity against established human cancer cell lines in a solid phase enzyme-linked immunosorbent assay - (ELISA). Tumor cells were removed from flasks with 0.25% trypsin, plated in microtiter plates (Costar, Cambridge, MA) at 50,000 cells per well, and incubated overnight at 37°C (5% $CO_2$). The cells were fixed with 0.15% glutaraldehyde for 15 minutes, washed with 0.1M borate-saline buffer, pH 8.5, containing 0.1% bovine serum albumin, and stored at 4°C until used. The ELISA was performed using the avidin-biotin immunoperoxidase system as described in the manufacturer's protocol (Vector, Labs, Burlingame, CA) incorporating either hybridoma supernatant fluids, ascites, or purified Mabs. O-phenylenediamine-hydrogen peroxide - (Abbott Laboratories, Chicago, IL) was used as the color indicator. Color development was quantitated by a Multiskan MC (Flow Laboratories, McLean, VA) and reactivity was defined as three standard deviations above the values obtained from a control Mab, specific for human alpha-fetoprotein (UMVA-AFP-02; E. Arfman et al., Fed. Proc., 42, 676 - (1983)), hereafter referred to as AFP-02.

The cell-binding ELISA was also adapted to screen for Mab reactivity against suspension cells and lymphocytes. Briefly, 200,000 cells were added to each well of the screening plate and attached to the well bottom using poly-1-lysine by the method of E. Kedar et al., J. Immunol., 112, 2058 (1977).

## C. Purification of Monoclonal Antibodies

Ascitic fluid samples from the BALB/c mice were centrifuged at 100,000 x g for 25 minutes at 4°C to remove lipid and cellular debris. An immunoglobulin fraction was precipitated from the supernatant by the addition of anhydrous sodium sulfate over 1-2 hours at room temperature with constant stirring to a final concentration of 20% - (w/v). The mixture was stirred for an additional 45 minutes and allowed to stand at room temperature overnight. The precipitated immunoglobulin was collected by centrifugation at 15,000 x g for 25 minutes at room temperature and dialyzed overnight at 4°C against 0.02 M phosphate buffer, pH 8.0, containing 0.02% $NaN_3$. Albumin and proteases were largely removed by passage of the sample over a CM Affi-Gel Blue column (BioRad Laboratories, Richmond, CA) equilibrated in the same buffer. Fractions containing the immunoglobulins were pooled, concentrated, and dialyzed against 0.02 M Tris-HCl buffer, pH 7.2, containing 0.02% $NaN_3$ using a Pro-Di-Con system (Bio-Molecular Dynamics, Beaverton, OR). Aliquots were applied to a DEAE Affi-Gel Blue column - (BioRad Laboratories) equilibrated in the same buffer at 4°C. After elution of unbound components, non-IgG proteins were typically eluted with 0.02 M Tris-HCl buffer, pH 7.2, containing 0.008 M NaCl and 0.02% $NaN_3$. Monoclonal antibody IgG was eluted isocratically with 0.02 M Tris-HCl buffer, pH 7.2, containing 0.018 M to 0.040 M NaCl and 0.02% $NaN_3$. Fractions of 1 ml were collected and absorbance at 280 nm was monitored. Appropriate fractions were pooled, concentrated, and dialyzed against PBS. Mab purification was assessed by sodium dodecylsulfate polyacrylamide gel electrophoresis using 10% gels. Gels were silver stained [D. Sammons, et al., Electrophoresis, 2, 135 (1981)], dried, and stored for reference. Using this protocol, Mab yields ranged from 50-75% and their purity consistently exceeded 90%. The contaminants were usually limited to traces of albumin and higher molecular weight components.

## D. Hybridomas Produced

Three highly-reactive monoclonal antibodies, designated as A6H, C5H, and D5D were isolated. The Mabs were characterized and employed to localize carcinoma xenografts as described hereinbelow. These Mabs, hereinafter referred to as A6H, C5H, and D5D, respectively, are of the IgG, subclass, express the kappa light chain, and when passaged in vivo, yielded from 2-6 mg of immunoglobulin per ml of ascitic fluid. The hybridoma cell lines which produce A6H (designated UMVA-RCC-A6H), C5H (designated UMVA-RCC-C5H) and D5D (designated UMVA-RCC-D5D) have been deposited with the American Type Culture Collection, Rockville, MD, USA, and have been assigned accession nos. ATCC Nos. HB 8803, HB 8801 and HB 8802, respectively. The hybridoma cell lines have been found to be stable producers for at least one year, are easily recovered from frozen storage, and remain mycoplasma-free. Mab A6H resulted from a tandem immunization schedule using five different human fetal kidney homogenates; and Mabs C5H and D5D resulted from a 5-month, 6-injection immunization of the RCC cell line 7860.

## Example 2 -Characterization of Antibodies

### A. Reactivity with Tissue Culture Cells

Mouse ascites fluid produced following injection of mice with each of the three hybridomas of the present invention and from the AFP control hybridoma was collected and adjusted to 1 mg IgG per ml. Each Mab was titered against a panel of 36 established human cell cultures using the cell binding ELISA system described in Ex. 1(C)(i), hereinabove. The results are summarized in Table I. The sources of the human tumor cell lines which were included in the cell-binding ELISA panel summarized in Table I are given in Table II, following.

## Table I

### Titration of Mabs against assorted
### human tumor cell lines

|  | Lowest Mab concentration (ng/ml) yielding a positive reaction* | | | |
|---|---|---|---|---|
|  | A6H | C5H | D5D | AFP-02 |

**CELL LINES**
**Renal Cell Carcinoma**

|  | A6H | C5H | D5D | AFP-02 |
|---|---|---|---|---|
| 769P | 25 | 50 | 25 | 5,000 |
| 1072F | 5 | 50 | 25 | 1,000 |
| 1934J | 25 | 50 | 25 | 1,000 |
| 7860 | 5 | 500 | 1 | 5,000 |
| A704 | 5 | 50 | 5 | 1,000 |
| ACHN | 1 | 50 | 25 | 1,000 |
| CAK1-1 | 5 | 25 | 5 | 500 |
| CAK1-2 | 5 | 50 | NR[a] | 500 |
| Pastor | 1 | 100 | 1 | 5,000 |
| SK-RC6 | 25 | 500 | 25 | 10,000 |
| SK-RC7 | 5 | 50 | 25 | 500 |
| TK-10 | 5 | 100 | 25 | 500 |
| TK-164 | 1 | 25 | NR | 1,000 |
| TK-204 | 1 | 25 | 1 | 100 |

### Bladder Carcinoma

| | | | | |
|---|---|---|---|---|
| 253J | NR | 50 | NR | 5,000 |
| 486P | NR | 500 | NR | 5,000 |
| 575A | 500 | 50 | NR | 10,000 |
| 639V | NR | 500 | NR | 5,000 |
| 647V | NR | 500 | NR | 1,000 |
| 5637 | NR | NR | NR | 500 |

### Prostatic Carcinoma

| | | | | |
|---|---|---|---|---|
| DU-145 | NR | 25 | NR | 500 |

### Melanoma

| | | | | |
|---|---|---|---|---|
| SK-Mel-5 | NR | 1 | NR | 500 |
| SK-Mel-22 | NR | NR | NR | 500 |

### Breast Carcinoma

| | | | | |
|---|---|---|---|---|
| MCF-7 | NR | ‹100 | NR | 500 |
| SK-BR-3 | 500 | 500 | NR | 5,000 |

### Lung Carcinoma

| | | | | |
|---|---|---|---|---|
| A-427 | NR | NR | NR | 500 |
| SK-Mes-1 | NR | NR | NR | 500 |

### Squamous Cell Carcinoma

| | | | | |
|---|---|---|---|---|
| UM-SCC-1 | NR | 100 | NR | 1,000 |
| UM-SCC-2 | NR | 500 | NR | 5,000 |

## Colon & Bowel Carcinoma

| | | | | |
|---|---|---|---|---|
| 1976L | 5 | 500 | NR | 500 |
| HT-29 | 50 | 500 | NR | 1,000 |
| SW-48 | NR | NR | NR | 5,000 |

## Testicular Carcinoma

| | | | | |
|---|---|---|---|---|
| 833K | NR | NR | NR | 1,000 |
| 1156Q | 100 | 500 | NR | 500 |
| 1411H | NR | NR | NR | 50 |

[a](NR=Nonreactive)

*Three S.D. above the O.D. of control Mab AFP-02 (indicator of nonspecific binding).

## Table II

### Source of Cell Lines[a]

| Cell Line | Source |
|---|---|

## Renal Cell Carcinoma

| | |
|---|---|
| 1934J | Dr. Michael Agrez, Mayo Clinic |
| ACHN | American Type Cell Culture Collection |
| CAK1-2 | American Type Cell Culture Collection |
| Pastor | Dr. DeKernian, Wadsworth and Brentwood Medical Center, Los Angeles, CA |
| SK-RC6 | Dr. Neil Bander, Sloan Kettering |
| SK-RC7 | Dr. Neil Bander, Sloan Kettering |

## Bladder Carcinoma

T-24      Dr. Carol O'Toole, University of
Tennessee

## Prostatic Carcinoma

DU-145      Dr. George Wright, Eastern Virginia
Medical School, Norfolk, VA

## Melanoma

SK-Mel-5      Dr. Thomas Carey, University of Michigan
SK-Mel-22      Dr. Thomas Carey, University of Michigan

## Breast Carcinoma

MCF-7      Dr. Clarence McGrath, Michigan Cancer
Foundation
SK-BR-3      American Type Cell Culture Collection

## Lung Carcinoma

A-427      American Type Cell Culture Collection
SK-Mel-1      American Type Cell Culture Collection

## Squamous Cell Carcinoma

UM-SCC-1      Dr. Thomas Carey, University of Michigan
UM-SCC-2      Dr. Thomas Carey, University of Michigan

## Colon & Bowel Carcinoma

| | |
|---|---|
| HT-29 | American Type Cell Culture Collection |
| SW-48 | American Type Cell Culture Collection |

a Represents cell lines other than those which were established at the University of Minnesota.

The data summarized in Table I are presented as the lowest Mab concentration (ng/ml) which results in an absorbance reading at least three standard deviations above that obtained employing the negative control Mab. These results illustrate that monoclonal antibody D5D is the most restricted, reacting with 12 of 14 RCC cell lines but no other human tumor lines. No reactivity against red blood cells (RBC) or lymphocytes was noted. Antibody A6H was also substantially restrictive, reacting with all 14 RCC lines but with few other (5/22): 1 of 7 transitional cell carcinoma of the bladder cell lines, 1 of 2 breast carcinoma cell lines, 2 of 3 colon and bowel cell cultures, and 1 of 3 testicular carcinomas. No reactivity was observed against RBC or lymphocytes. The antibody C5H reacted with the majority of the human tumor cell lines (29/36) including all of the RCC cultures. RBC and lymphocyte reactivity was not observed. With the exception of C5H, the Mabs were highly reactive, often yielding a positive response at 1 ng/ml against the RCC cell lines.

B. Immunopathological Evaluation of the Antibodies

Surgical specimens were preserved in the following manner. Tissues were returned to the laboratory in ice-cold saline within one hour after excision. Necrotic, connective, and fatty tissue was removed and the specimen was cut into pieces not exceeding 2 mm/side. Each specimen was preserved by at least two of the following three methods: (a) tissue pieces were held on ice while 2 ml of 2-methylbutane (isopentane; Eastman Kodak Co., Rochester, NY) was placed into 4 ml screw-top glass vials. The vials were placed in a rack half-submerged in liquid nitrogen for one to three minutes to freeze the isopentane. Rubber-lined caps were attached, and the vials were stored at -80°C; (b) tissues were stored in OCT embedding medium (Miles Laboratories, Naperville, IL). Specimens were submerged in 1 ml of OCT contained in wells of a polyvinylchloride 24-well tissue culture plate, frozen on dry ice, and stored at -80°C; (c) tissues were placed in cold buffered formalin and paraffin-embedded tissue blocks were prepared by conventional techniques.

For analysis, samples were removed, sectioned with a cryostat (frozen specimens) or microtome - (paraffin-embedded specimens) and stained (with immunofluorescence or immunoperoxidase) using the following procedures. Immunofluorescent tissue staining was performed on 4-um cryostat sections obtained from either fresh or stored tissue after fixation in either acetone or 0.15% glutaraldehyde in PBS for ten minutes and washing three times for three minutes each in PBS, pH 7.4. The sections were then incubated with 50 ul of purified Mabs (2 ug/ml) or fresh culture supernatants for thirty minutes. The sections were washed, overlaid with FITC-labeled rabbit anti-mouse IgG, and washed again. Goat anti-rabbit-FITC conjugate was added and the slides were incubated for an additional 45 minutes at room temperature. The sections were washed and mounted in wet Gelvatoll 20-30 - (Monsanto, St. Louis, MO). All sections were evaluated using an Olympus BH-2 epi-fluorescence and phase microscope.

Immunoperoxidase staining was performed on both fresh frozen and paraffin-embedded tissue as described by Wordinger in Manual of Immunoperoxidase Techniques, R. Wordinger, G. W. Miller, D. S. Nicodemus, eds., American Society of Clinical Pathologists Press, Chicago, Ill. (1983), using as a secondary antibody goat anti-mouse IgG labeled with horseradish peroxidase and 3',3'-diaminobenzidine tetrahydrochloride as a color indicator. Sections were counterstained with hematoxylin. In both procedures, controls consisted of AFP-02 and other IgG, Mabs that were nonreactive with RCC tissue cultures at the same concentrations as the Mabs under evaluation.

Representative immunohistochemical reactivity profiles of the three tumor-specific Mabs are shown in Table III. While the data in Table III represent an analysis of immunofluorescent staining patterns using cryostat sections of fresh frozen specimens, the Mabs were also tested against paraffin-embedded tissues by the immunoperoxidase technique. With the exception of C5H, which does not react with . paraffin-embedded tissues, the profiles were similar, although the degree of reactivity was usually somewhat weaker.

## Table III

### Reactivity of Monoclonal Antibodies with Human Tumor, Normal and Fetal Tissues

| ANTIBODY: | A6H | | | C5H | | | D5D | | |
|---|---|---|---|---|---|---|---|---|---|
| Reactivity Group[*]: | 0 | 1-2+ | 3-4+ | 0 | 1-2+ | 3-4+ | 0 | 1-2+ | 3-4+ |
| **TISSUE** | | | | | | | | | |
| **Cancer** | | | | | | | | | |
| Kidney (RCC) | 2 | 9 | 4 | 2 | 14 | 1 | 5 | 12 | 0 |
| Kidney (Non-RCC) | 1 | 0 | 0 | 0 | 1 | 0 | 1 | 0 | 0 |
| Kidney (TCCB[a] metastases) | 1 | 0 | 0 | 0 | 1 | 0 | 1 | 0 | 0 |
| Lung (RCC metastases) | 1 | 1 | 0 | 0 | 2 | 0 | 0 | 2 | 0 |
| Bladder (TCCB) | 5 | 0 | 0 | 1 | 6 | 0 | 7 | 0 | 0 |
| Prostate | 4 | 0 | 0 | 3 | 4 | 0 | 7 | 0 | 0 |
| Colon | 6 | 6 | 0 | 3 | 8 | 0 | 11 | 0 | 0 |
| Lung | 1 | 0 | 0 | 1 | 0 | 0 | 1 | 0 | 0 |
| Testicle | 3 | 0 | 0 | 1 | 6 | 0 | 6 | 0 | 0 |
| Lipoblasts | 0 | 0 | 0 | 1 | 0 | 0 | 1 | 0 | 0 |
| Penile | 2 | 0 | 0 | 2 | 0 | 0 | 2 | 0 | 0 |
| Pancreatic | 1 | 0 | 0 | 0 | 1 | 0 | 1 | 0 | 0 |

Normal

| | | | | | | | | | |
|---|---|---|---|---|---|---|---|---|---|
| Kidney | 0 | $11^b$ | 0 | 0 | $11^b$ | 0 | 11 | 0 | 0 |
| Lung | 6 | 0 | 0 | 6 | 0 | 0 | 6 | 0 | 0 |
| Colon | 5 | 0 | 0 | 5 | 0 | 0 | 5 | 0 | 0 |
| Bladder | 4 | 0 | 0 | 4 | 0 | 0 | 4 | 0 | 0 |
| Prostate | 4 | 0 | 0 | 4 | 0 | 0 | 4 | 0 | 0 |
| Liver | 4 | 0 | 0 | 4 | 0 | 0 | 4 | 0 | 0 |
| Stomach | 3 | 0 | 0 | 3 | 0 | 0 | 3 | 0 | 0 |
| Testis | 4 | 0 | 0 | 4 | 0 | 0 | 5 | 0 | 0 |
| Spleen | 3 | 0 | 0 | 3 | 0 | 0 | 3 | 0 | 0 |

Fetal

| | | | | | | | | | |
|---|---|---|---|---|---|---|---|---|---|
| Kidney | 0 | $8^b$ | 0 | 0 | $8^b$ | 0 | 8 | 0 | 0 |
| Spleen | 6 | 0 | 0 | 6 | 0 | 0 | 6 | 0 | 0 |
| Lung | 5 | 0 | 0 | 5 | 0 | 0 | 5 | 0 | 0 |
| Liver | 4 | 0 | 0 | 7 | 0 | 0 | 7 | 0 | 0 |
| Colon | 5 | 0 | 0 | 5 | 0 | 0 | 5 | 0 | 0 |
| Stomach | 3 | 0 | 0 | 3 | 0 | 0 | 3 | 0 | 0 |
| Bladder | 1 | 0 | 0 | 2 | 0 | 0 | 2 | 0 | 0 |
| Pancreas | 2 | 0 | 0 | 2 | 0 | 0 | 2 | 0 | 0 |
| Thymus | 4 | 0 | 0 | 4 | 0 | 0 | 4 | 0 | 0 |
| Bone Marrow | 4 | 0 | 0 | 4 | 0 | 0 | 4 | 0 | 0 |

aTCCB=Transitional cell carcinoma of the bladder.
bSee text for discussion of selective renal staining pat-
terns.

\* 0 = none; 1-2+ = weak to moderate; 3-4+ = strong.

In addition to the tissues listed in Table III, there were three opportunities to study both the primary RCC and its metastasis. The tissues were immunofluorescently stained with. A6H, C5H, and D5D (Table IV). The intensity of the staining and the pattern of staining were similar in each series.

Table IV

RCC Primary and Metastatic
Immunohistochemical Reactivities

| | Immunofluorescent Grading of Mab Reactivity | | |
|---|---|---|---|
| RCC TISSUE SPECIMENS | A6H | C5H | D5D |

Primary(P);  Metastatic(M)

| | | A6H | C5H | D5D |
|---|---|---|---|---|
| 1133 | P | ++ | + | ++ |
| 1133 | M | ++ | + | ++ |
| 1166 | P | ++ | ++ | − |
| 1166 | M | + | + | − |
| 3441 | P | + | ++ | + |
| 3441 | M | + | ++ | + |

The results of all the tissue immunohistochemical studies illustrate patterns of reactivity which are similar to those obtained in the cell-binding ELISA and provide additional information concerning the expression of the recognized antigens. D5D remained the most restricted, reacting with most RCC surgical specimens (14/19 primary and metastatic samples) but not with any other human tumors or non-renal normal and fetal tissues in the panel (0/110 tissue samples), and exhibiting an apparent absence of any consistent reactivity with adult or fetal kidney (0/19 tissue samples). (D5D on rare occasions has reacted in a +/- to 1+ level with the Bowman's capsule.) A6H identifies an epitope shared by RCC (14/17 specimens), 6 of 12 colon carcinomas, and the proximal tubules of normal and fetal kidneys. Breast carcinoma tissue sections were negative (0/1 specimen). No other cancer or normal tissues expressed the A6H reactive antigen. C5H reacts well with RCC (17/19 tissues) and most other human tumor sections (27/37 samples), the glomerulus of normal and fetal kidney, but no other non-tumor tissues (0/70 tissue sections).

Other noteworthy observations include the absence of A6H or D5D reactivity with (a) a non-RCC renal tumor (i.e., an oncocytoma) and (b) a metastatic transitional cell carcinoma of the bladder which was located within the kidney.

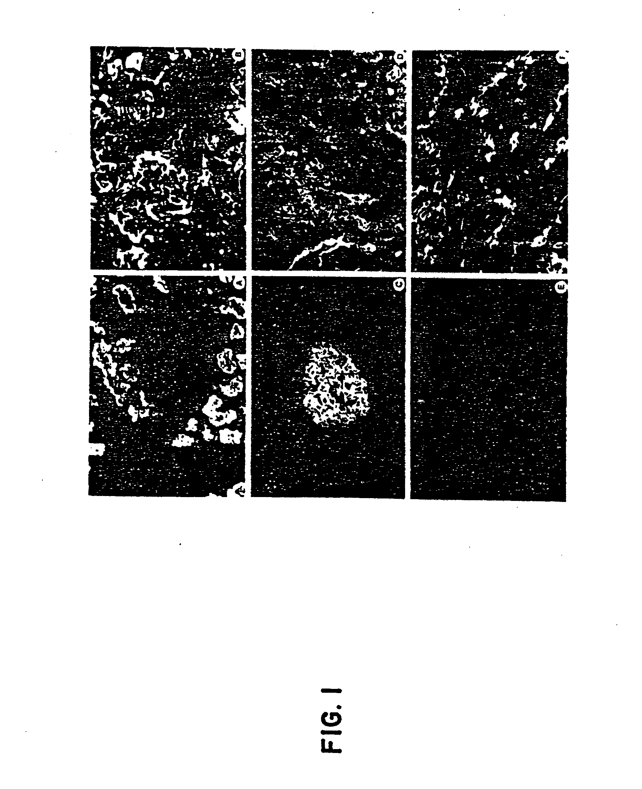

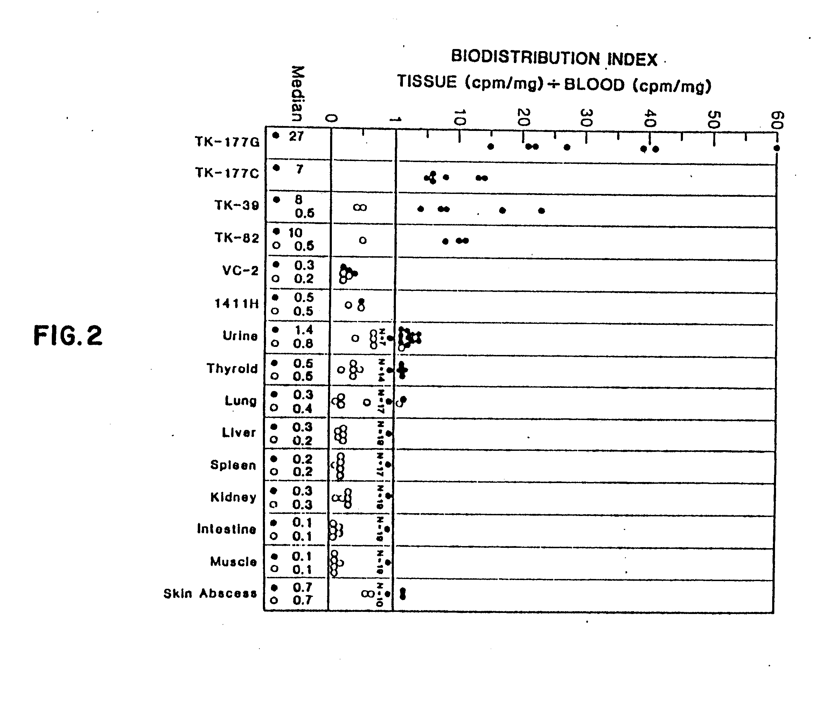

FIGURE 1 is a photographic composite depicting the typical staining patterns observed with Mabs A6H (A&B), C5H (C&D) and D5D (E&F) on normal kidney (A, C and E) and RCC (B, D and F) sections. The specimens were flash frozen in isopentane and 4 um sections were prepared. Sections were fixed with glutaraldehyde and thoroughly washed. The sections were then incubated with 50 ul of purified Mab (2 ug/ml) for 30 minutes, washed and overlaid with FITC-labeled rabbit antimouse IgG and FITC-labeled goat anti-rabbit antibody. Pronounced proximal tubular staining is obtained with A6H as shown in FIGURE 1A and the glomerular staining obtained with C5H is shown in FIGURE 2C. D5D exhibits no visible normal renal reactivity as shown in FIGURE 1E. All three Mabs stained the RCC sections.

Example 3 -Tumor Detection and Imaging

A. Radiolabeling of Monoclonal Antibodies

Purified A6H, C5H, D5D or the control IgG, - (UMVA-AFP-22) was radiolabeled with either 125-I or 131-I using the chloramine T procedure. The Mab (70-150 ug) was mixed with 1-2 mCi of Na-(125-I) or Na(131-I) and 5 mg chloramine T in 0.1 M phosphate buffer, pH 7.2, for 20-30 seconds after which tyramine was added to stop the reaction. The iodinated immunoglobulin was separated from excess reactants by gel filtration (Bio-Gel P-6DG; Bio-Rad Laboratories). Specific activities of the radiolabeled Mab ranged from 5-20 uCi/ug. Each radiolabeled Mab preparation was evaluated for immunologic activity. An aliquot of the preparation (approximately $10^6$ cpm) was added to replicate tubes containing $10^5$ cells of an RCC cell line - (7860), previously shown to be highly reactive with A6H. The tubes were incubated at room temperature with periodic mixing. After one hour, the cells were pelleted by centrifugation, and the amount of radiolabel bound to the cells determined and expressed as the percent of total radiolabeled Mab added to the tubes. This value, representing the immunologically reactive portion of the radiolabeled preparation, generally ranged from 50-70%, although on occasion low immunological activity - (<30%) was noted. These preparations of low activity were not utilized in the studies presented hereinbelow.

B. Human Tumor Xenografts

Human RCC xenografts were established subcutaneously in nude mice from fresh surgical specimens and maintained in the colony by serial passage. The six RCC xenografts utilized in these studies were prepared from cell lines established at the University of Minnesota and designated TK-39, TK-82, TK 161, TK 163, TK-177C, and TK-177G. These xenografts closely resemble the histology of their original human tumor.

Control human tumor xenografts incorporated in this study were VC-2, an endometrial carcinoma provided by Dr. P.G. Satyaswaroop, Milton S. Hershey Medical Center, Philadelphia, PA); a melanoma (Sk-Mel-28; Sloan Kettering, N.Y.), a human testicular carcinoma (1411 H) and a lymphoma (1179 L) from the University of Minnesota.

C. Biodistribution of Radiolabeled Mabs

Mice undergoing RIS received 20-40 uCi (2-4 ug) of 131-I radiolabeled Mab, whereas those used solely for biodistribution studies received 1 to 2 uCi (0.1-0.2 ug) of the radiolabeled Mab (125-I). The biodistribution of the radiolabel was determined immediately following scanning.

In order to determine the quality of localization, several parameters using eight different tissues were calculated: (a) the tumor-blood (T:B) ratio, (b) a Tumor:Tissue or T:T ratio with particular emphasis on the accretion of radiolabel in the liver and spleen, (c) the ratio between RCC preferential Mab accretion and non-specific Mab accretion in each xenograft, (d) the percent of injected dose in the xenograft on a per gram basis and (e) the percent of radiolabel within the xenograft compared to the whole body at the time of imaging.

Figure 2 is a graphic depiction of the specificity indices (SI) or T:B ratios for the first 59 mice entered into the RIS study.

As illustrated in FIGURE 2, the T:B ratios (i.e. biodistribution indexes) post-injection of radiolabeled A6H ranged from 4 to 60 in the RCC xenografts. In the non-RCC xenografts and most of the normal tissues the specificity indexes were <1 for all of the labelled antibodies. Of twenty-three thyroid samples, four resulted in biodistribution indexes for A6H of 1-2, as did one of the eighteen lung specimens. In contrast, AFP-22 did not show enhanced localization in any of the xenografts or normal tissues tested. (None of the mice bearing RCC xenografts TK-177C or TK-177G received AFP-22.) The slightly increased biodistribution indexes (T:B ratios) on day 3 when compared to day 2 were insignificant overall.

Since normal tissues normally contain less radiolabel per unit weight than blood, T:T ratios tend to yield considerably higher values than T:B ratios. For example, as presented in Table V, the selectivity of A6H for the RCC xenografts is represented by median T:T ratios that often exceeded about 10-20 and in eleven instances exceeded 100, whereas AFP-22 did not exhibit any selectivity among the xenografts or tissues involved in these investigations. Alternatively, in animals bearing both a Mab specific xenograft (e.g. RCC for A6H) and a nonspecific xenograft, a tumor specificity index can be calculated based on the retention of radiolabel per unit weight in each xenograft, e.g., specific tumor (cpm/mg)/nonspecific tumor (cpm/mg). In the three animals bearing both TK-82 and VC-2, A6H yielded tumor specificity indexes of 24, 33 and 37.

Table VI summarizes data derived from a random selection of 30 mice from the study summarized on Fig. 2, comparing T:B, T:T (liver and spleen) and percent injected dose values measured following injection radiolabelled A6H, C5H and D5D.

## Table V

### Enhanced Accumulation of A6H in RCC Xenografts

| XENOGRAFT: | Tumor:Tissue Ratio[a] | | | | | | | |
|---|---|---|---|---|---|---|---|---|
| | TK-39 | | TK-82 | | TK-177C | | TK-177G | |
| TISSUE | Median | Range | Median | Range | Median | Range | Median | Range |
| Blood | 8 | 4- 24 | 10 | 8- 11 | 7 | 5- 14 | 27 | 15- 60 |
| Intestine | 84 | 26-181 | 75 | 70- 92 | 68 | 22-144 | 270 | 84-600 |
| Kidney | 31 | 14-147 | 35 | 26- 37 | 32 | 11- 60 | 90 | 42-250 |
| Liver | 42 | 13- 92 | 37 | 30- 44 | 37 | 15- 55 | 108 | 57-231 |
| Lung | 32 | 12-118 | 12 | 7- 20 | 42 | 17- 85 | 146 | 44-410 |
| Muscle | 44 | 39-118 | 97 | 65-110 | 51 | 27- 85 | 135 | 105-353 |
| Spleen | 60 | 20- 97 | 49 | 37- 55 | 30 | 22- 72 | 116 | 63-300 |
| Skin Abscess | 12 | 5- 21 | | | 10 | 5- 19 | 32 | 21- 63 |

a=RCC xenograft (cpm/mg)/Tissue (cpm/mg). Quantitation of radiolabel accretion occurred on day 2 or 3 post i.v. injection of 125-I and 131-I radiolabeled A6H (1-40 uCi/dose; specific activities of approximately 10 uCi/ug).

Table VI

BIODISTRIBUTION PARAMETERS

Range of Biodistribution Values[a]

| Mab | T:B | T:T(Liver) | T:T(Spleen) | % Injected dose/g |
|-----|-----|-----------|-------------|-------------------|
| A6H | 8 - 40 | 17 - 137 | 22 - 117 | 17 - 145 |
| C5H | 2 - 14 | 5 - 22 | 6 - 27 | 9 - 32 |
| D5D | 1 - 10 | 2 - 17 | 3 - 17 | 3 - 12 |

a) Ten animals from each Mab study were randomly selected for presentation. The table contains data from mice bearing different RCC xenografts.

A further measurement used in evaluating the potential of an antibody for RIS is to calculate the percent of radiolabeled antibody in the xenograft per unit weight of tissue as a function of injected dose. Of the animals receiving a 1 uCi dose of 125-I-radiolabeled A6H and whose RCC xenografts were removed on day 2, five determinations were made: TK-177G (28% and 40% of injected dose per gram), TK-177C (7% and 9%), and TK-39 - (13%). In mice sacrificed on day 3, the values were uniformly higher: TK-177G (84%, 95% and 140%) and TK-177C (21% and 51%).

Mice with Staphylococcal skin abscesses were involved in the final series of biodistribution studies. Two of the thirteen specimens exhibited marginally elevated T:B ratios of 1.2 and 1.4. All of the other abscessed skin samples had T:B ratios of <1, although the actual values were higher than those usually found in normal tissues. These data demonstrate that the IgG, Mabs A6H and AFP-22 may accumulate slightly in areas of acute tissue inflammation, but the levels rarely exceed the concentration of radiolabeled antibody in the blood.

D. Radioimmunoscintigraphy

Two RIS studies were performed in conjunction with the Mab biodistribution investigations. In the first, three mice bearing both TK-177C and TK-177G xenografts were intravenously injected with 20 uCi of 131-I A6H.

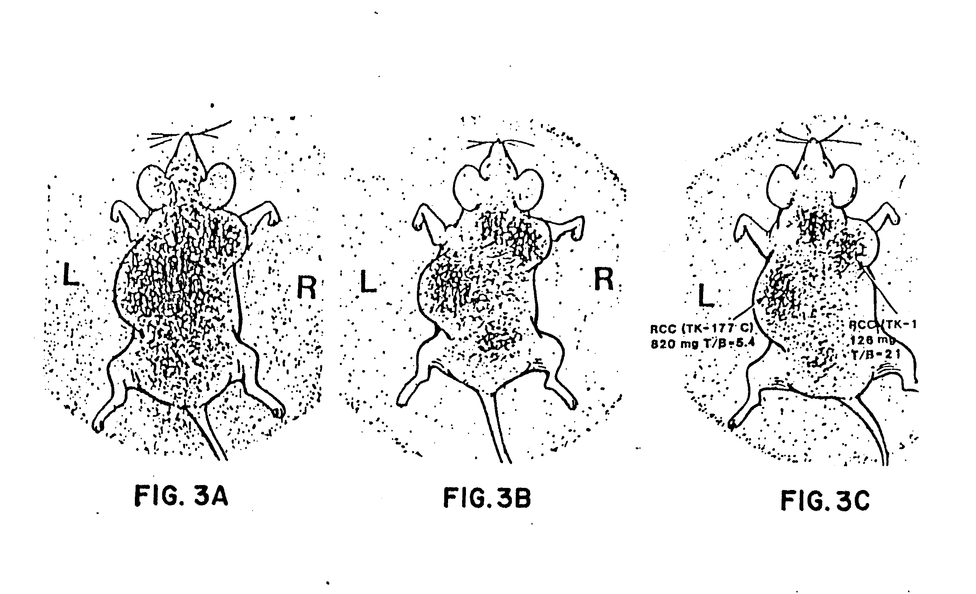

FIGURE 3 depicts the label density in one mouse as measured by serial scintigraphy performed at five hours (scan A), one day (scan B), and two days (scan C) following the injection. Immediately following the last scan, T:B ratios were determined and these are expressed in scan C along with the weight of each xenograft. Background subtraction techniques were not used.

Although scintigraphic distinction of the RCC xenografts was marginal at five hours, clear images were observed by day 2. FIGURE 3C also indicates the xenograft size and the T:B ratios of 5.4 (TK-177C) and 21 (TK-177G). These ratios are among the lowest observed for these two xenografts as set forth on Table V.

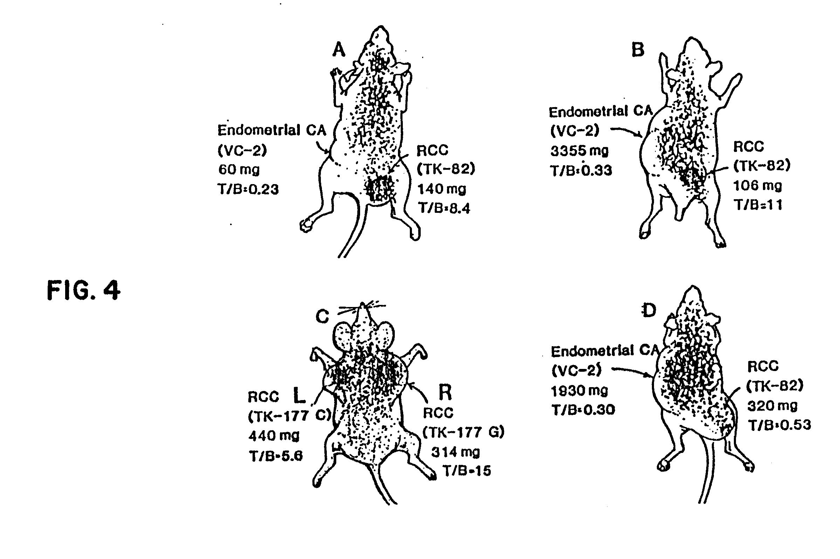

The second RIS series consisted of eight mice bearing either two RCC xenografts or one RCC xenograft and a second non-RCC xenograft. Four of the RIS scans are depicted in FIGURE 4, along with details of xenograft size and T:B ratios. Mice received either 131-I radiolabeled AFP-22 (N=3) FIGURE 4D or A6H (N=5) FIGURES 4A-C (1-40 uCi/dose; 10 uCi/ug specific activity). Imaging was performed on day 2 followed by biodistribution analysis. As previously shown (FIG. 3), A6H always localized in the RCC xenografts providing T:B ratios of at least about 4, whereas AFP-22 consistently yielded T:B ratios of <1. In the RIS series depicted in FIG. 4, all non-RCC xenografts and normal tissue T:B ratios were <1. Consistent with these data, the RIS scans detailed the specific localization of A6H to the RCC xenografts but not to non-RCC xenografts. For example, in scan B, A6H shows unquestionable discrimination between a small RCC xenograft (TK-82, 106 mg) and the larger endometrial carcinoma xenograft control - (VC-2, 3355 mg). The respective T:B ratios were 11 and 0.33. Also, in this animal the specificity index was 33. In a similar manner, the fact that AFP-22 did not highlight any of the tumor xenografts by RIS was supported by xenograft T:B ratios of <1.

It was not necessary to utilize background subtraction techniques to visualize the RCC xenografts localized with A6H. In fact, there was no significant accumulation of A6H in the liver or the spleen by RIS and this was verified by consistently low (<1) T:B ratios in these organs. Also, there appeared to be a correlation between the intensity of the RIS scan and the T:B ratio -TK-177G was clearly the easiest to visualize and had the highest median T:B ratio of 27. In addition, TK-177G exhibited the highest percentage of radiolabel dose per unit weight. Finally, RIS was sufficiently sensitive to detect a 60 mg TK-39 xenograft which had a biodistribution index of 23.

The detection of approximately 40 mg tumors appears to be the lower limit of sensitivity for the subcutaneous site employing the above method, since the initial implant is always at least 25 mg. Therefore, the sub-renal capsule (src) implantation technique was employed to establish 6 RCC xenografts from implants of 2 mg with a >90% take rate. The doubling time ranged from 8-12 days at this site compared to 15-30 days when the grafts were implanted subcutaneously. In preliminary src RIS studies using the three labelled Mabs, successful imaging was achieved of xenografts ranging in size from 7-28 mg with T:B ratios ranging from 1.2-42 and xenograft:kidney ratios of 14-68 (N-24). The percent injected dose per gram ranged from 10->500%.

E. Radioimmunotherapy (RIT) of RCC Xenografts Using 131 I-A6H:

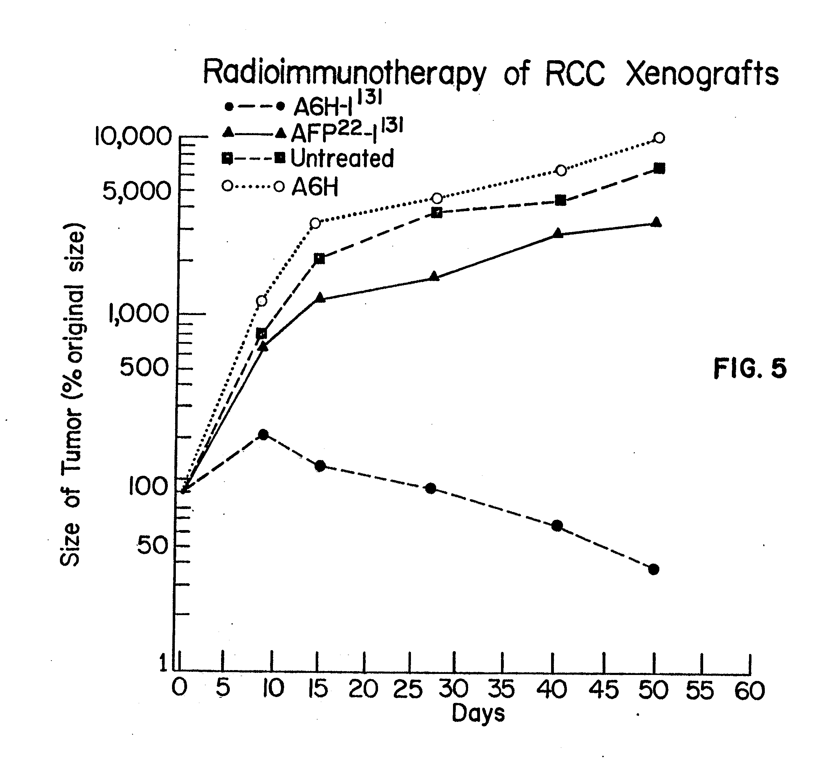

Preliminary RIT studies were initiated in four experiments each lasting up to 150 days. At the time of injection (day 0), the xenografts TK-82 or TK-177 were approximately 50-100 mg in size. Each series consisted of four groups of animals: one received 100 uCi (10 uCi/ug specific activity) 131-I radiolabeled A6H injected i.v.; a second received unlabeled A6H (10 ug i.v. dose), a third group received an equivalent dose of 131-I radiolabeled Mab specific for AFP (AFP-22) and the last group received no treatment. Therapy was repeated on day 20. Periodically, tumor size was quantitated, a size index was calculated (w x d x h), and the results expressed as a percentage of the tumor size on day 0. The results summarized in Figs. 5A-D showed a dramatic inhibition of tumor growth with RCC radiolabeled Mabs. The number of animals entered into each group is indicated in parentheses at the end of the survival curves.

In addition to the size index, survival data was also calculated for these four series of experiments and are presented in Table VII.

## Table VII

## SUMMARY OF RADIOIMMUNOTHERAPY SURVIVAL DATA[a]

| Treatment | Total No. Entered | No. Presumed Indicental Death[b] | No. Presumed Tumor Death[c] | |
|-----------|-------------------|----------------------------------|-----|-----------------------|
| | | | No. | Mean Survival (days) |
| None | 16 | 4 | 11 | 67 |
| Non-labeled A6H | 8 | 3 | 5 | 61 |
| $^{131}$I-AFP-22 | 15 | 2 | 13 | 74 |
| $^{131}$I-A6H | 17 | 12 | 0 | >150 |

[a] Analysis of survival in tumor bearing nude mice is difficult since these animals often die of "incidental" causes during the course of study. Percent change in tumor size is probably a better parameter for assessing the effects of therapy.

[b] May include death from infection, anesthesia or unknown causes - limited to mice bearing tumors <1000% of original size.

[c] Death from any cause when tumor is >1000% of original size.

The mean survival among the three control groups ranged from 61-74 days with all showing tumor progression >10 fold (N=29). In contrast, none of the 16 animals treated with two doses of the radiolabelled A6H showed progression, although some animals in each group died of infection or anesthesia while we were estimating tumor size. Mean survival in the group treated with the radiolabelled A6H was more than 150 days. One mouse received only one dose of the radiolabelled A6H; after initial tumor arrest some progression was observed at day 75. At sacrifice (day 90) a few viable tumor cells were found in the nodule. By contrast, the two mice that received two doses of the labelled A6H were also sacrificed at this time - (Figure 5B -group II) and both nodules showed only fibrosis with no evidence of viable tumor cells. Dosimetry calculations indicated that each 100 uCi injection of the RCC Mab A6H results in the delivery of approximately 5500 rads to the RCC xenografts over a three week period with total body irradiation being less than 300 rads. This calculation is encouraging because it indicates tumoricytal dosages are being administered specifically to the tumor while normal tissues receive tolerable irradiation. These studies represent the first successful treatment of RCC xenografts by any modality.

DISCUSSION

Compared to other radioimmunolocalization studies, the labeled Mabs of the present invention demonstrate unusually high and specific T:B ratios. For example, the accumulation of radiolabeled A6H within the four RCC xenografts ranged from 4 to ≥ 60 times that in the blood and from 13 to >200 times that in the liver, while none of the RCC tumors demonstrated specific uptake of the control Mab AFP-22. As shown on Table VI, antibodies C5H and D5D have exhibited somewhat lower but nevertheless very high T:B ratios. These antibodies have also provided highly discriminatory images. Insignificant control Mab accretion suggests that the high T:B ratios obtained with A6H, C5H and D5D were not due to physical trapping of immunoglobulin in the tumor tissue. Furthermore, it is unlikely that accumulation of the Mabs within the RCC xenografts was due to an inflammatory effect since the amount of the Mab found in acutely inflamed skin abscesses was usually less than in the circulation, and histological evaluations of several RCC xenografts showed insignificant inflammation in the tumors.

In the present RIS studies, clear tumor resolution was obtained consistently without using subtraction techniques even for the smallest RCC xenograft in the series, which was 0.1 cm in diameter and 7.0 mg in weight under the sub-renal capsule. Indeed, this small xenograft accumulated 5.0% of the injected dose, or >500% on a per gram basis. Also, in animals bearing both a RCC and a non-RCC xenograft, RIS distinguished the two tumors and biodistribution analysis of the xenografts yielded a median tumor specificity index of 33. All these data indicate that the localization of the present Mabs is an antigen-mediated event which is highly preferential for RCC.

Furthermore, when the present data is converted to percent injected dose of radioactivity per gram of tumor, A6H continues to show exceptionally high levels of selective incorporation. For example, in one series of tests, A6H yielded a mean of 34% of injected dose/g on day 2 and 106% on day 3 in TK-177G. Also, with A6H, over 0.1% of the total body radioactivity is present in the RCC per mg of tissue at day 2. In comparison, Pimm and Baldwin, in Eur. J. Cancer Clin. Oncol., 20, 515 - (1984) using an osteogenic sarcoma preferential Mab, reported: (a) T:B ratios ranging from 0.6-2.9, - (b) 8.9% of the injected dose/g on day 2, and (c) 0.03% of whole body radioactivity/mg in the xenograft. These values are comparable to those obtained with other tumor preferential antibodies that have shown preferential biodistribution but lack the level of discrimination necessary for the detection of small tumors by RIS. For example, see S. Larson et al., J. Nucl. Med., 24, 123 (1983). Thus, the present Mabs appear to show exceptional localization by these parameters as well as by T:B ratios.

In summary, hybridoma techniques have been employed to produce new cell lines which yield monoclonal antibodies which bind strongly to a variety of renal cell carcinomas, while not binding substantially to normal tissue. Two of the hybridomas yield antibodies A6H and D5D which are highly specific for renal cell carcinoma, reacting with 100% and with more than 85% of the human RCC cell lines, respectively, to which the antibodies were exposed. These antibodies are substantially non-reactive with other human tumor cell lines. A third monoclonal antibody, C5H, is 100% reactive with RCC cell lines and substantially reactive with other human tumor cell lines. When labeled with radioactive iodine, all three antibodies became highly concentrated in human RCC xenografts, thus permitting the tumors to be effectively visualized by radioimmunoscintigraphy, without the need for background subtraction techniques. Furthermore, injections of radiolabeled (131-I) antibody A6H caused a significant reduction in RCC xenograft tumor masses. Thus it is expected that the monoclonal antibodies of the present invention will be useful in the diagnosis, imaging and radiotherapy of RCC in human patients in clinical settings.

The present invention has been described with reference to various specific and preferred embodiments and techniques. However, it should be understood that many variations and modifications will become apparent to those skilled in the art from the foregoing description and may be made while remaining within the spirit and scope of the invention. The invention described and claimed herein is not to be limited in scope by the cell lines deposited, since the deposited embodiments are intended as illustrations of specific aspects of the invention. Any other hybridoma cell lines, including naturally-arising or intentionally produced mutations of the present hybridomas, which produce functionally equivalent monoclonal antibodies are within the scope of this invention.

**Claims**

1. A method for detecting and imaging a renal cell carcinoma comprising injecting a human subject with a monoclonal antibody produced by a hybridoma cell line selected from the group consisting of UMVA-RCC-C5H, ATCC No. HB 8801; UMVA-RCC-D5D, ATCC No. HB 8802; UMVA-RCC-A6H, ATCC No. HB 8803 and the mutations thereof which are able to bind to said carcinoma cells while not substantially binding to normal cells; wherein said monoclonal antibody is radiolabeled with a radioisotope capable of detection with a photoscanning device; localizing the antibody in the renal cell carcinoma; and determining the radioactivity of the carcinoma-bound monoclonal antibody by photoscanning the subject with the photoscanning device so that the carcinoma is detected and visualized.

2. The method of Claim 1 wherein the carcinoma is visualized by scintigraphy.

3. The method of Claim 1 wherein the monoclonal antibody is labeled with a radioisotope of a halogen atom.

4. The method of Claim 1 wherein the monoclonal antibody is labeled with a radioisotope selected from the group consisting of Iodine 125, Iodine 131, Iodine 123, Indium 111, Ruthenium 97, Copper 67 and Technicium 99.

5. The method of Claim 1 wherein the monoclonal antibody does not substantially localize in human non-renal cell carcinomas.

6. The method of Claim 1 wherein the ratio of the amount of antibody in the tumors to the amount of antibody in the blood of the subject is at least about 4.

7. A method. of tumor radioimmunotherapy which comprises introducing into a human subject having a renal cell carcinoma a radiotherapeutically-effective amount of a monoclonal antibody produced by a hybridoma cell line selected from the group consisting of UMVA-RCC-C5H, ATCC No. HB 8801; UMVA-RCC-D5D, ATCC No. HB 8802; UMVA-RCC-A6H, ATCC No. HB 8803 and the mutations thereof, which is able to bind to said carcinoma cells while not substantially binding to normal cells; wherein said monoclonal antibody is radiolabeled with a radioisotope; and localizing said monoclonal antibody in said carcinoma.

8. The method of Claim 7 wherein the radioisotope is selected from the group consisting of Iodine 131, Yttrium 90, Gold 199, Palladium 100, Copper 67, Bismuth 217 and Antimony 211.

9. A monoclonal antibody of subclass IgG₁, produced by a hybridoma formed by fusion of cells from a mouse myeloma line and spleen cells from a mouse previously immunized with a plurality of human fetal kidney homogenates, which antibody:
(a) expresses the kappa light chain,
(b) is about 100% reactive with human renal cell carcinoma cell lines,
(c) is substantially non-reactive with human non-renal cell carcinoma cell lines, and
(d) reacts with the proximal tubules of normal and fetal human kidney tissue but is substantially non-reactive with other normal human tissue.

10. The monoclonal antibody of Claim 9 which is produced from a hybridoma formed by fusion of P3-NSI-Ag4-1 or P3X3-Ag8.653 mouse myeloma cells with spleen cells obtained from a BALB/c mouse previously immunized in a tandem immunization schedule with a plurality of phenotypically similar, genotypically-dissimilar human fetal kidney homogenates.

11. A monoclonal antibody of subclass IgG₁, produced by a hybridoma formed by fusion of cells from a mouse myeloma line and spleen cells from a mouse previously immunized with a human renal cell carcinoma cell line, which antibody:
(a) expresses the kappa light chain,

(b) reacts with more than 85% of human renal renal cell carcinoma cell lines,
(c) does not react with non-renal cell human carcinoma cell lines, and
(d) does not react with normal human tissue.

12. The monoclonal antibody of Claim 9 which is produced from a hybridoma formed by fusion of P3-NSI-Ag4-1 or P3X3-Ag8.653 mouse myeloma cells with spleen cells obtained from a BALB/c mouse previously immunized with the human renal cell carcinoma line 7860.

13. A monoclonal antibody of class IgG₁, produced by a hybridoma formed by fusion of cells from a mouse myeloma line and spleen cells from a mouse previously immunized with a human renal cell carcinoma cell line, which antibody:
(a) expresses the kappa light chain;
(b) is about 100% reactive with human renal cell carcinoma cell lines;
(c) is substantially-reactive with non-renal cell carcinoma cell lines; and
(d) reacts with the glomerulus of normal and fetal human kidney tissue but does not react with other normal human tissue.

14. A monoclonal antibody of Subclass IgG₁, produced by a hybridoma formed by fusion of cells from a mouse myeloma line and spleen cells from a mouse previously immunized with the human renal cell carcinoma line 7860.

15. A hybridoma cell line having the characteristics of UMVA-RCC-A6H, ATCC No. HB 8803, and the mutations thereof, formed by a process comprising fusing P3-NSI-Ag4-1 or P3X3-Ag8.653 mouse myeloma cells with spleen cells obtained from a BALB/c mouse previously immunized in a tandem immunization schedule comprising the sequential injection of the mouse with a plurality of phenotypically similar, genotypically dissimilar human fetal kidney homogenates, which hybridoma cell line produces a monoclonal antibody against a human renal carcinoma cell line.

16. The hybridoma cell line of Claim 15 wherein five different human fetal kidney homogenates were employed in the immunization - schedule.

17. A hybridoma cell line having the characteristics of UMVA-RCC-C5H, ATCC No. HB 8801, and the mutations thereof, formed by a process comprising fusing P3-NSI-Ag4-1 or R3X3-Ag8.653 mouse myeloma cells with a murine spleen cell line obtained from a BALB/c mouse immunized with the renal cell carcinoma cell line 7860, which hybridoma cell line produces a monoclonal antibody against a human renal carcinoma cell line.

18. A hybridoma cell line having the characteristics of UMVA-RCC-D5D, ATCC No. HB 8802, and the mutations thereof, formed by a process comprising fusing P3-NSI-Ag4-1 or P3X3-Ag8.653 mouse myeloma cells with a murine spleen cell line obtained from a BALB/c mouse immunized with the renal cell carcinoma cell line 7860, which hybridoma cell line pro duces a monoclonal antibody against a human renal carcinoma cell line.

19. The monoclonal antibody produced by the hybridoma cell line of Claim 15.

20. The monoclonal antibody produced by the hybridoma cell line of Claim 17.

21. The monoclonal antibody produced by the hybridoma cell line of Claim 18.

22. The radiolabeled monoclonal antibody prepared by a process comprising binding a radioisotope to the monoclonal antibody of Claims 9, 11, 13, 19, 20 or 21.

23. The radiolabeled antibody of Claim 22 wherein a halide selected from the group consisting of Na(I-125), K(I-125), Na(I-131), K(I-131) and mixtures thereof is reacted with the monoclonal antibody in the presence of chloramine T.

24. A pharmaceutical composition comprising the radiolabeled monoclonal antibody of Claim 22 and a pharmaceutically-acceptable liquid carrier therefor.

FIG. I

FIG.2

BIODISTRIBUTION INDEX
TISSUE (cpm/mg) ÷ BLOOD (cpm/mg)

Median

TK-177G • 27
TK-177C • 7
TK-39 • 8 / 0.5
TK-82 • 10 / ○ 0.5
VC-2 • 0.3 / ○ 0.2
1411H • 0.5 / ○ 0.5
Urine • 1.4 / ○ 0.8 N=7
Thyroid • 0.5 / ○ 0.5 N=14
Lung • 0.3 / ○ 0.4 N=17
Liver • 0.3 / ○ 0.2 N=19
Spleen • 0.2 / ○ 0.2 N=17
Kidney • 0.3 / ○ 0.3 N=19
Intestine • 0.1 / ○ 0.1 N=19
Muscle • 0.1 / ○ 0.1 N=19
Skin Abscess • 0.7 / ○ 0.7 N=10

0 210 970

FIG. 3A

FIG. 3B

FIG. 3C

FIG. 4

A

Endometrial CA
(VC-2)
60 mg
T/B=0.23

RCC
(TK-82)
140 mg
T/B=8.4

B

Endometrial CA
(VC-2)
3355 mg
T/B=0.33

RCC
(TK-82)
106 mg
T/B=11

C

RCC L
(TK-177 C)
440 mg
T/B=5.6

RCC R
(TK-177 G)
314 mg
T/B=15

D

Endometrial CA
(VC-2)
1930 mg
T/B=0.30

RCC
(TK-82)
320 mg
T/B=0.53

0 210 970

Radioimmunotherapy of RCC Xenografts

FIG. 5

0 210 970